# EUROPÄISCHE PATENTSCHRIFT

(11) **EP 3 468 637 B1**
(45) Veröffentlichungstag und Bekanntmachung des Hinweises auf die Patenterteilung: **07.01.2026**
(21) Anmeldenummer: 17735386.9
(22) Anmeldetag: 12.06.2017
(51) Int. Cl.: A61M 1/16, B01D 63/02

(54) **GAS-AUSTAUSCH-EINHEIT UND VERFAHREN ZUM HERSTELLEN EINER GAS-AUSTAUSCH-EINHEIT**
GAS EXCHANGE UNIT AND METHOD FOR PRODUCING A GAS EXCHANGE UNIT
UNITÉ D'ÉCHANGE GAZEUX ET PROCÉDÉ DE PRODUCTION D'UNE UNITÉ D'ÉCHANGE GAZEUX

(30) Priorität: 10.06.2016 DE 102016007105; 30.08.2016 DE 102016010398
(43) Veröffentlichungstag der Anmeldung: 17.04.2019
(73) Patentinhaber: Hemovent GmbH, 52076 Aachen (DE)
(72) Erfinder: MARSEILLE, Oliver, 52066 Aachen (DE); SCHMITZ, Bernhard, 53937 Schleiden (DE)
(74) Vertreter: Sattler de Sousa e Brito, Clara
(86) Internationale Anmeldenummer: PCT/EP2017/000685
(87) Internationale Veröffentlichungsnummer: WO 2017/211460

(56) Entgegenhaltungen:
- WO-A1-99/52621
- DE-A1- 102011 052 188
- GB-A- 2 533 027
- JP-A- S61 143 075
- US-A1- 2002 057 989
- US-B1- 6 454 999

## Beschreibung

Die vorliegende Erfindung betrifft eine Gas-Austausch-Einheit zur Verwendung in der extrakorporalen Membranoxygenierung (ECMO) oder dem extrakorporalem life support (ECLS) und ein Verfahren zur Herstellung einer solchen Gas-Austausch-Einheit.

Herz-Lungen-Maschinen ersetzen die lebenswichtigen Kreislauffunktionen der Blutförderung und des Gasaustauschs (O2 Eintrag und CO2 Entfernung aus dem Blut) während einer Herzoperation. Abgeleitet davon können Herz-Lungen-Maschinen auch eingesetzt werden, um Patienten mit Herz- oder Lungeninsuffizienz über Tage zu stabilisieren. Hierbei spricht man von extrakorporaler Membranoxygenierung (ECMO) oder extrakorporalem live support (ECLS).

Die derzeit verwendeten ECMO-Oxygenatoren basieren auf dem Design der Oxygenatoren, wie sie in der Herzchirurgie verwendet werden. In der Regel werden die lediglich offenporigen Membranfasern durch Membranen mit geschlossenen Diffusionsmembranen ersetzt. Vor diesem Hintergrund basieren die Spezifikationen und die Auslegung auf den Anforderungen der Herzchirurgie.

Bekannte Oxygenatoren sind z.B. in JP S61 143075 oder WO 99/52621 offenbart.

Aufgabe der vorliegenden Erfindung ist es, die aus dem Stand der Technik bekannten Oxygenatoren insbesondere für ECMO und ECLS Anwendungen zu verbessern.

Dies wird durch eine Gas-Austausch-Einheit mit einem Hohlfasermodul erreicht, wobei die Gastauschercharakteristik des Hohlfasermoduls anpassbar ist. So kann bei sich ändernden Anforderungen an die Gastauschercharakteristik vor oder während des Betriebs diese bedarfsgerecht adaptiert werden. Insbesondere kann eine Adaption während der Produktion erfolgen. Das Hohlfasermodul kann insbesondere eine Faserplatte sein.

Unter einer Anpassung der Gastauschercharakteristik einer Gas-Austausch-Einheit ist, die Veränderung des CO2 Abtrag und/oder des 02 Eintrag oder die Veränderung des Verhältnisses der zwei Werte zueinander zu verstehen. Dies kann auf verschiedene Weise erzielt werden.

Unter einer Anpassung der Gastauschercharakteristik ist insbesondere eine Änderung der wirksamen Oberfläche oder der effektiven Faserlänge zu verstehen. Eine Änderung der effektiven Faserlänge kann beispielsweise durch das In-Reihe oder In-Serie-Schalten der Fasern erzielt werden. So kann es beispielsweise von Vorteil sein eine lange effektive Faserlänge durch eine gegenströmige Beschickung vorzusehen, da so der Sauerstoffverbrauch reduziert werden kann.

Eine Anpassung der Gastauschcharakteristik kann auch durch eine Veränderung der Flussgeschwindigkeit des Gases in den Fasern des Hohlfasermoduls erfolgen. Diese Flussgeschwindigkeit des Gases kann durch ein Druckgefälle von außen eingestellt werden. Über die Länge der Faser nimmt das Konzentrationsgefälle für CO2 ab, d.h. nach den ersten Fick'schen Gesetz sinkt der Übertrag von CO2. Wird jedoch die Strömgeschwindigkeit erhöht, wird dieser Effekt schwächer, d.h. der Übertrag von CO2 wird erhöht. Dies hat jedoch einen negativen Einfluss auf die zu verwendende Sauerstoffmenge.

Vorteilhaft ist hierbei, wenn ein Hohlfasermodul ganz oder teilweise vom Gasstrom zu- oder abschaltbar ist. So kann die Gastauschercharakteristik innerhalb eines Hohlfasermoduls durch Änderung der wirksamen Oberfläche angepasst werden.

Besonders vorteilhaft ist hierbei, wenn die Gas-Austausch-Einheit auf der Gasseite eine Blende, insbesondere einen Schieber und / oder einen Drehschieber und / oder eine Drossel aufweist, wobei die Blende so angeordnet ist, dass Faserbereiche nicht durchströmt geschaltet werden können.

Erfindungsgemäß sind Faserbereiche des Hohlfasermoduls unterschiedlich mit Gas beschickbar.

Dadurch können die Faserbereiche in Abhängigkeit vom blutseitigen Gastransferbedarf (CO2 und 02) von unterschiedlichen Gasmengen und - Zusammensetzungen durchflossen werden und so auch der Sauerstoffverbrauch reduziert werden.

Weiter von Vorteil ist, wenn die Gas-Austausch-Einheit eine Überstromkanalvorrichtung aufweist. Durch diese kann die unterschiedliche Beschickung der Faserbereiche durch verschiedenen Druckgefälle erfolgen. Die Überstromkanalvorrichtung kann mehrere Kammern aufweisen, die durch Überstromkanäle verbunden sind. Insbesondere kann eine solche Vorrichtung auf beiden Seitenflächen, auf denen das Gas in das Hohlfasermodul einströmt, angebracht sein.

Von Vorteil ist, wenn die Überstromkanalvorrichtung verstellbare Überstromkanäle aufweist. Dadurch kann das Strömungsbild in der Überstromkanalvorrichtung angepasst werden und so an unterschiedliche Benutzungszustände der Gas-Ausstausch-Einheit adaptiert werden. Dies kann beispielsweise durch einen Schieber erfolgen, der an einer Verstellvorrichtung angeordnet ist.

Weiterhin von Vorteil ist, wenn das Hohlfasermodul eine Abdeckung aufweist. Dadurch kann auf der Seitenfläche auf der das Gas einströmt der pneumatische Widerstand erhöht werden. Es können eine oder mehrere Abdeckungen aufgebracht werden. Auch so kann eine unterschiedliche Beschickung der Faserbereiche des Hohlfasermoduls erzielt werden.

Zudem von Vorteil ist, wenn die Abdeckung einen pneumatischen Widerstandsgradienten aufweist. Unterschiedliche Dicke oder unterschiedlichen Materialien (unterschiedliche Porosität). So können auch kontinuierliche Übergänge in der Beschickung erfolgen. In diesem Fall verschwimmen die Bereichsgrenzen.

Weiter von Vorteil und unabhängig erfinderisch ist, wenn dem Hohlfasermodul eine gasseitige Befeuchtungs- und Wärmevorrichtung vorgeschaltet ist. Hierdurch kann das Gas vor Eintritt in das Hohlfasermodul erwärmt werden. So wird das Gas mit Wasserdampf gesättigt und weist eine Temperatur über der Bluttemperatur auf. Im Hohlfasermodul gibt das Gas Wärme ab und ein Teil des Wasserdampfs kondensiert und gibt die Kondensationsenergie an das Blut ab. Das Kondensat kann unterhalb des Wärmetauschers gesammelt werden.

Das Hohlfasermodul kann trommelförmig sein. Dies erlaubt einen platzsparenden Aufbau und bietet in der Produktion Vorteile.

Bei einer trommelförmigen Anordnung ist von Vorteil, wenn das Durchmesser-zu-Längenverhältnis des Hohlfasermoduls > 1,5 ist. Hierdurch ergeben sich bei gleicher wirksamer Oberfläche kürzere Fasern. Der Partialdruckabfall des Sauerstoffs wird auf der Innenseite reduziert und so wird der Oxygenator bzgl. der CO2 Transferrate effektiver als herkömmliche Produkte.

Weiter von Vorteil ist, wenn in dem Hohlfasermodul eine blutundurchlässige Schicht von innen nach außen spiralförmig angeordnet ist. So wird der Blutstrom in eine gewisse Richtung gelenkt und der Durchströmungsweg kann so kontrolliert werden. Insbesondere kann der Durchströmungsweg so verlängert werden. Hierbei kann der Blutstrom von innen nach außen oder von außen nach innen oder diagonal nach außen oder nach innen führbar sein. Dies hat den Vorteil, dass verschiedene geometrische Anordnungen möglich sind.

Von Vorteil kann sein, wenn die Gas-Austausch-Einheit zwei oder mehr Hohlfasermodule aufweist. Hierdurch kann die Gastauschercharakteristik vor oder während des Betriebs der Gas-Austausch-Einheit durch Änderung der wirksamen Oberfläche in den einzelnen Hohlfasermodulen angepasst werden. Bei mehreren Hohlfasermodulen ist dies insbesondere durch unterschiedliche Anordnung der Hohlfasermodule möglich. So kann die Langzeitstabilität, Blutkompatibilität und Leistungsfähigkeit vor dem Hintergrund der abweichenden Indikation bei ECMO und ECLS verbessert werden.

Erfindungsgemäß sind die Hohlfasermodule unterschiedlich mit Gas beschickbar.

Dadurch können die unterschiedlichen Hohlfasermodule in Abhängigkeit vom blutseitigen Gastransferbedarf (CO2 und 02) von unterschiedlichen Gasmengen und -Zusammensetzungen durchflossen werden und so auch der Sauerstoffverbrauch reduziert werden.

Weiter von Vorteil ist, wenn die Gas-Austausch-Einheit eine Überstromkanalvorrichtung aufweist. Durch diese kann die unterschiedliche Beschickung der Hohlfasermodule durch verschiedene Druckgefälle erfolgen. Die Überstromkanalvorrichtung kann mehrere Kammern aufweisen, die durch Überstromkanäle verbunden sind. Insbesondere kann eine solche Vorrichtung auf beiden Seitenflächen, auf denen das Gas in die Hohlfasermodule einströmt, angebracht sein.

Besonders von Vorteil ist, wenn die Hohlfasermodule mit einem oder mehreren Ventilen verbunden sind. Hierdurch kann die Durchströmrichtung der einzelnen Hohlfasermodule durch das Ventil gesteuert werden. So lassen sich die Gastransferraten auf die klinischen Indikationen abstimmen, indem über die Zusammen- oder Parallelschaltung der Module die CO2 Elimintaionsrate und der 02 Eintrag separat eingestellt werden können.

Auch kann die wirksame Gastauescheroberfläche bei Bedarf während der Therapie reduziert oder erweitert werden.

Auch ist es so möglich andere therapeutisch wirksame Gase einzubringen, indem einzelne Hohlfasermodule damit beschickt werden.

Besonders vorteilhaft ist, wenn die Hohlfasermodule im Gasstrom parallel oder in Serie schaltbar sind. Allein durch die unterschiedliche Schaltung kann so die Gastauschercharakteristik verändert werden. Es kann geregelt werden, ob neues unverbrauchtes Gas oder bereits einen Gasaustausch durchlaufenes Gas für ein weiteres Hohlfasermodul verwendet wird. Auch ist es möglich die Hohlfasermodule gezielt mit verschiedenen Gaszusammensetzungen (O2, CO2 und O2, 02 und NO usw.) zu beschicken. Insbesondere ist es möglich unterschiedliche Hohlfasermodule mit unterschiedlichen Gaszusammensetzungen zu beschicken.

Weiter können die Hohlfasermodule trommelförmig und konzentrisch ineinander angeordnet sein. Hierbei kann das Blut von innen nach außen oder von außen nach innen oder diagonal nach außen geführt werden. Auch in diesem Aufbau ist es möglich, dass in den Hohlfasermodulen eine blutundurchlässige Schicht von innen nach außen spiralförmig angeordnet ist. Hierdurch kann das Blut spiralförmig von innen nach außen oder von außen nach innen geleitet werden. Hierbei sind ein, zwei oder mehr Kanäle möglich.

Weiter von Vorteil ist, dass die Hohlfasermodule als Fasermatten ausgebildet sind und im Blutfluss hintereinander angeordnet sind. So können mehrere Hohlfasermodule in Stapelanordnung zu einer Gas-Austausch-Einheit verbunden werden. Die so verbundenen Hohlfasermodule können entsprechend unterschiedlich verschaltet verbunden werden und so miteinander kombiniert und mit Gas beschickt werden.

Von Vorteil ist, wenn der Faserverlauf einer Fasermatte zu dem Faserverlauf einer zweiten Fasermatte in einem Winkel angeordnet ist. Hierbei besonders von Vorteil ist, wenn der Winkel zwischen 10 ° bis 170 °, insbesondere 120 ° ist. Durch die Kreuzung kann ebenfalls die Gastauschercharakteristik und die Blutführung verbessert werden. Weiter von Vorteil ist, wenn das Durchmesser-zu-Längenverhältnis der Hohlfasermodule kleiner 1 ist. Auch hierdurch ergeben sich bei gleicher wirksamer Oberfläche kürzere Fasern mit den o.g Vorteilen.

Von Vorteil ist, wenn die Gas- Austausch-Einheit ein Heizelement aufweist. Dieses Heizelement kann elektrisch sein. Weiter ist es vorteilhaft, wenn es gasseitig in der Gas-Austausch-Einheit angeordnet ist. Dieses kann in einem Gehäuse angeordnet sein. Dadurch kann Wärme in das Blut eingebracht und eine Kondenzwasserbildung verhindert werden.

Von Vorteil ist es, wenn das Heizelement auf der Gaseinlassseite angeordnet ist um das Gas vor dem Austauschvorgang mit dem Blut aufzuheizen.

Das Heizelement kann stabförmig oder plattenförmig ausgeführt sein. Auch ist es möglich, dass das Heizelement durch einen auf einer Trägerstruktur aufgewickelten Draht realisiert wird oder mäandrierend gebogen ist.

Von Vorteil ist es, wenn das Heizelement Strukturen aufweist um die Austauschfläche zwischen Heizelement und Gas zu vergrößern. Diese können aus Rippen oder Blechen bestehen, um die das Gas herumgeführt wird. Bleche können Bohrungen aufweisen durch die das Gas strömt. Auch ist es denkbar, dass das Gas durch von außen beheizte Kanäle geführt wird.

Auch ist es vorteilhaft, dass zwei oder mehrere Heizelemente gasseitig hintereinander oder parallel angeordnet sind.

Von Vorteil ist es auch, dass ein stangen- oder plattenförmiges Heizelement von außen in die Gas-Austausch-Einheit eingesteckt und nach dem Einsatz an einer anderen Gas- Austausch-Einheit wiederverwendet werden kann.

Weiterhin von Vorteil ist, dass das Einbettmaterial zur Blutseite hin einen zylindrischen Abschluss bilden kann. Hierdurch ergibt sich eine homogene Strömungsverteilung, die niedrige Scherspannungen und gute Auswaschung hat und somit besonders blutschonend ist.

Ein weiterer Aspekt der Erfindung betrifft ein Verfahren zum Herstellen einer Gas-Austausch-Einheit, bei dem das Einbetten der Fasern in einem einzigen Produktionsschritt in einer Zentrifuge erfolgen kann

Erfindungsgemäß erfolgt in einem ersten Schritt eine automatisierte Herstellung der eines Hohlfasermoduls aus einer oder mehreren Fasermatten.

Aus mehreren Hohlfasermodulen wird dann modular in einem zweiten Schritt durch Zusammensetzen und Verpotten die Gas-Austausch-Einheiten mit unterschiedlicher wirksamer Oberfläche und effektiver Faserlänge erzeugt. Die Hohlfasermodule können dann zusätzlich vor Benutzung, bei Inbetriebnahme oder während des Betriebes ganz oder teilweise freigeschaltet oder mittels unterschiedlicher Schaltung mit Gas beschickt werden.

Die Erfindung wird im Folgenden anhand der Zeichnung näher erläutert. Hierbei zeigen die
- Figur 1: eine schematische Darstellung einer Gas-Austausch-Einheit mit zwei Hohlfasermodulen in Serienschaltung.
- Figur 2: eine schematische Darstellung einer Gas-Austausch-Einheit mit zwei Hohlfasermodulen in Parallelschaltung.
- Figur 3: eine schematische Darstellung einer Gas-Austausch-Einheit mit zwei Hohlfasermodulen, wobei eines zu- oder abgeschaltet werden kann.
- Figur 4: eine schematische Darstellung des Funktionsprinzips des Wärmeaustauschs in einem Hohlfasermodulen bei gasseitiger Vorschaltung einer Befeuchtungs- und Wärmevorrichtung.
- Figur 5 a: eine schematische Darstellung einer Gas-Austausch-Einheit mit zwei Hohlfasermodulen, die trommelförmig und konzentrisch ineinander angeordnet sind.

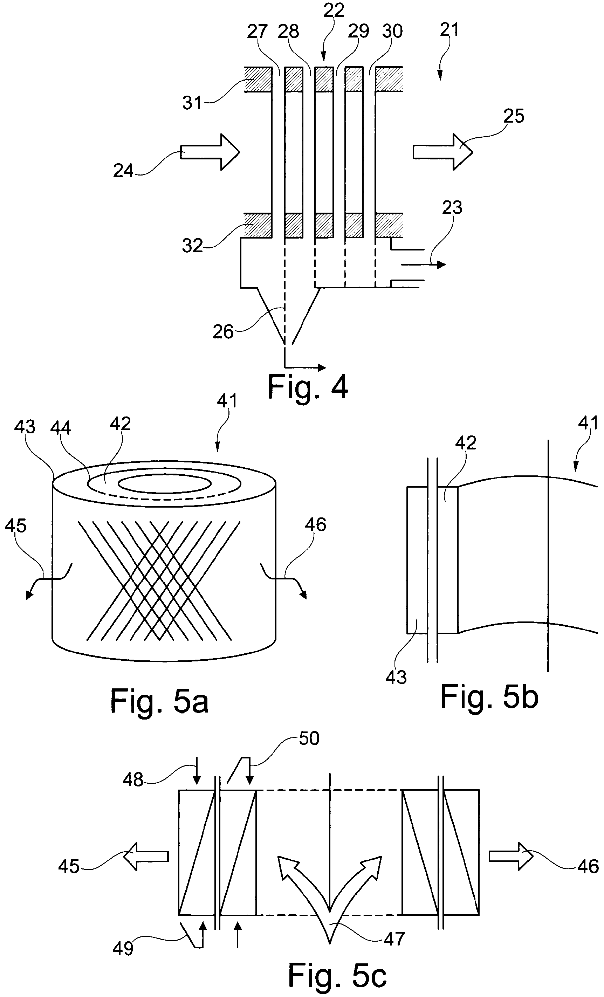

- Figur 5 b: einen Querschnitt aus der Gas-Austausch-Einheit in Figur 5.
- Figur 5 c: einen weiteren Querschnitt der Gasaustauscheinrichtung aus Figur 5, wobei sowohl die Luftdurchströmrichtung als auch die Blutstromrichtung schematisch anschaulich gemacht werden.

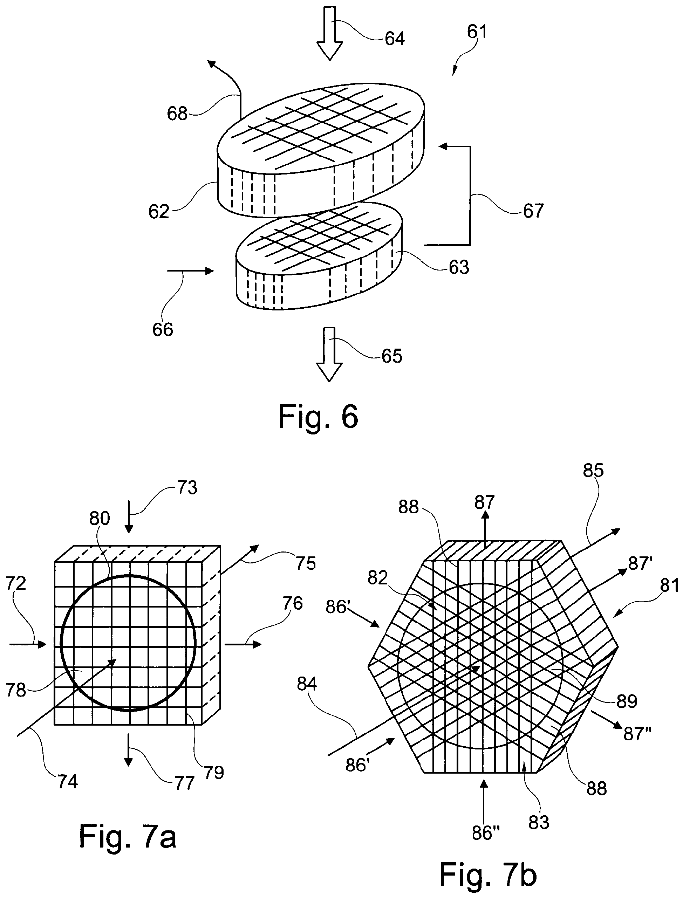

- Figur 6: eine schematische Darstellung einer Gas-Austausch-Einheit, wobei die Hohlfasermodule als Fasermatten ausgebildet sind.
- Figur 7 a: eine schematische Darstellung einer Gas-Austausch-Einheit aus Figur 6, wobei das Einbettmaterial zur Blutseite einen zylindrischen Abschluss bildet.
- Figur 7 b: eine schematische Darstellung einer Faseranordnung aus Figur 6, wobei die Fasermatten wechselseitig in einem Winkel von 120 ° gekreuzt angeordnet sind und das Einbettmaterial zur Blutseite hin zylindrisch ausgeführt ist.

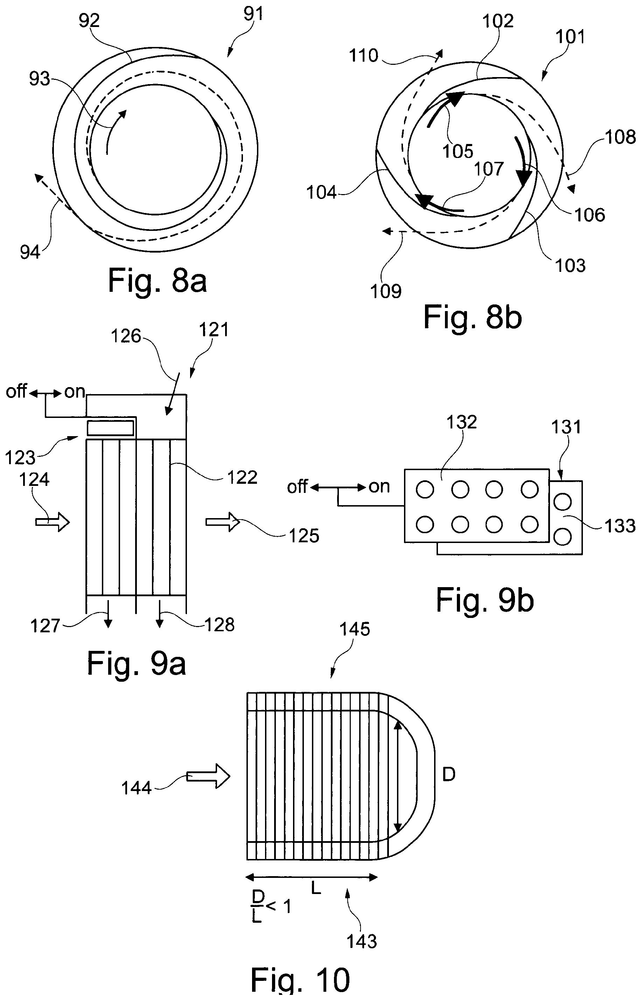

- Figur 8 a: eine schematische Darstellung einer Draufsicht auf eine Gas-Austausch-Einheit, in welcher die Hohlfasermodule trommelförmig sind und konzentrisch ineinander angeordnet sind und eine undurchlässige Schicht zur Blutleitung verwendet wird.
- Figur 8 b: eine schematische Darstellung einer ebenfalls trommelförmigen Hohlfasermodulanordnung, wobei durch eine undurchlässige Schicht mehrere Kanäle gebildet werden.
- Figur 9 a: eine schematische Darstellung einer Gas-Austausch-Einheit, wobei ein Teil der Gastauscherfasern stufenlos zu- oder abgeschaltet werden kann.
- Figur 9 b: eine schematische Darstellung eines Ventils zur Zu- und Abschaltung.
- Figur 10: eine schematische Darstellung einer Gas-Austausch-Einheit, wobei das Durchmesser-zu-Längenverhältnis der Hohlfasern > 1 ist.

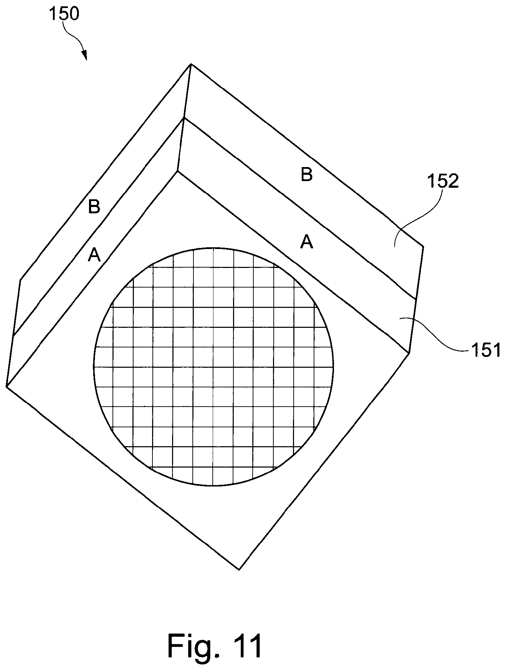

- Figur 11: eine schematische Darstellung einer Gas- Austausch-Einheit mit zwei Holfasermodulen, die als Fasermatten ausgebildet sind und hintereinander angeordnet sind

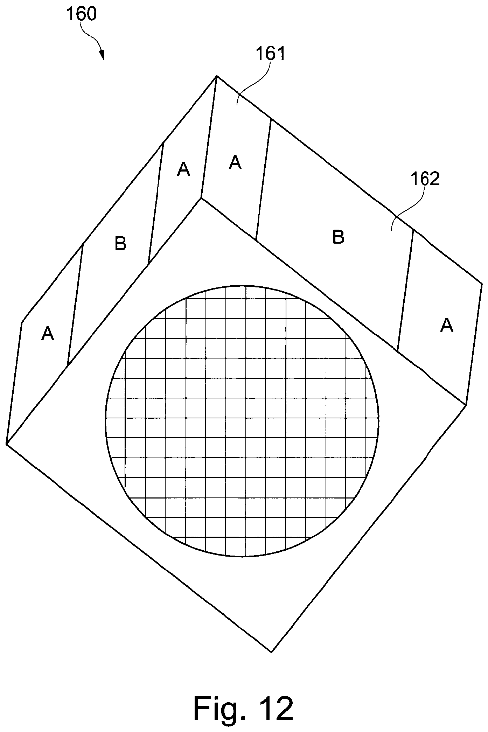

- Figur 12: eine schematische Darstellung einer Gas- Austausch-Einheit, die als Fasermatte ausgebildet ist, mit Faserbereichen, die nebeneinander angeordnet sind

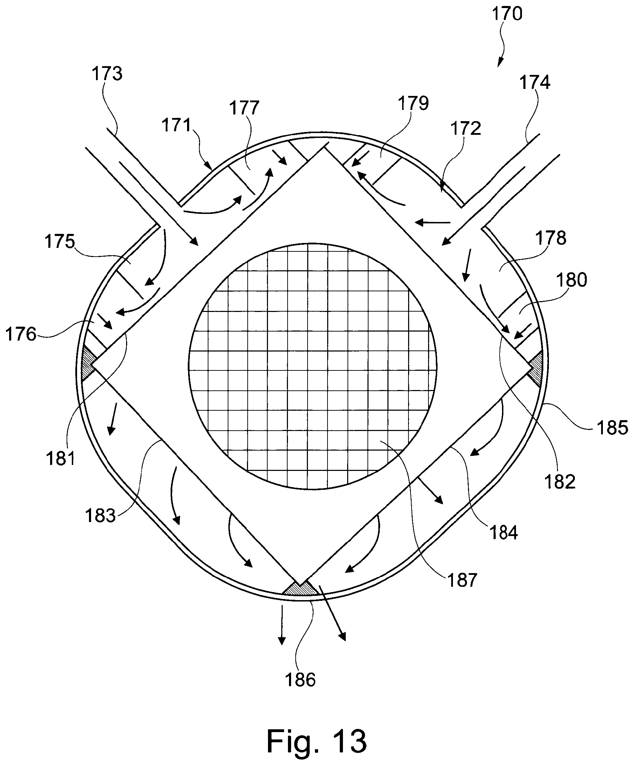

- Figur 13: eine schematische Darstellung einer Gas- Austausch-Einheit mit einer Überstromkanalvorrichtung

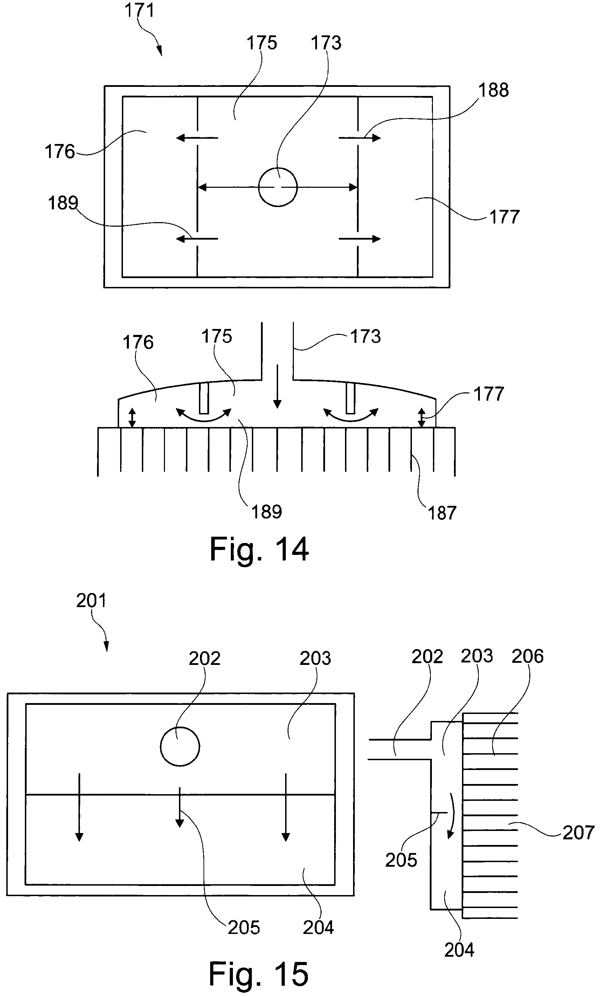

- Figur 14: eine schematische Darstellung der Anordnung des Zulaufs zu der Überstromkanalvorrichtung in einer Anordnung nach Figur 12 in Seitenansicht in Figur 14a) und im Ausschnitt eines Querschnittes in Figur 14b)
- Figur 15: eine schematische Darstellung der Anordnung des Zulaufs zu der Überstromkanalvorrichung in einer Anordnung nach Figur 11 in Seitenansicht in Figur 15a) und im Ausschnitt eines Querschnittes in Figur 15b)

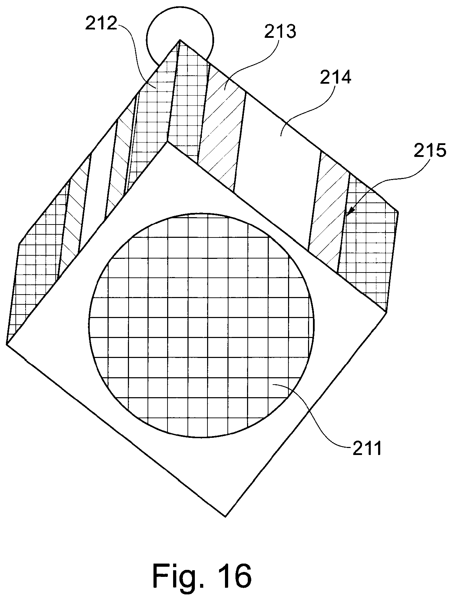

- Figur 16: eine schematische Darstellung einer Gasaustauscheinheit mit verschiedenen Auflagen zur pneumatischen Widerstandserhöhung

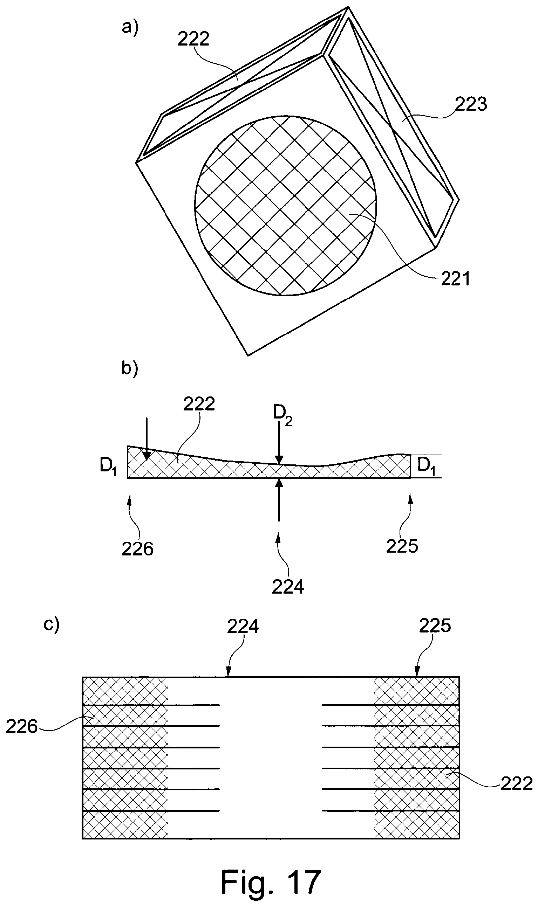

- Figur 17: eine schematische Darstellung einer Gasaustauscheinheit mit einer Auflage mit unterschiedlicher Stärke zur pneumatischen Widerstandserhöhung in Figur 17a), mit Seitenaufsicht auf die Auflage in Figur 17b) und Draufsicht auf die Auglage in Figur 17c)

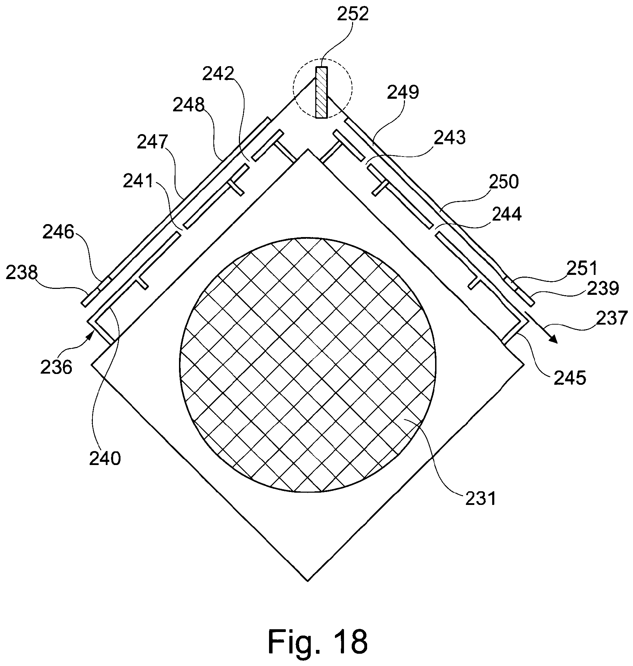

- Figur 18: eine schematische Darstellung einer Gasaustauscheinheit mit verstellbaren Überströmkanälen

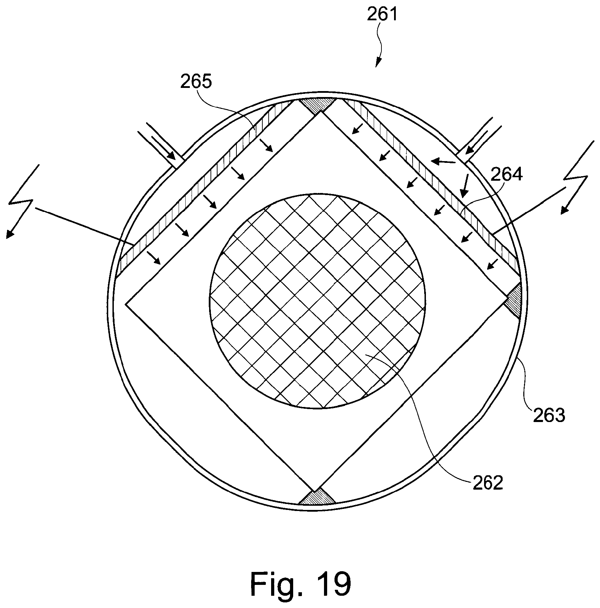

- Figur 19: eine schematische Darstellung einer Gasaustauscheinheit mit einem elektischen_{[OM1]} Heizelement

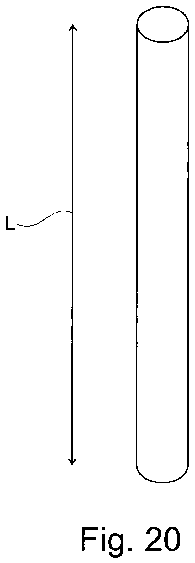

- Figur 20: eine schematische Darstellung eines Holfaser mit Angaben zur Geometrieabgrenzung

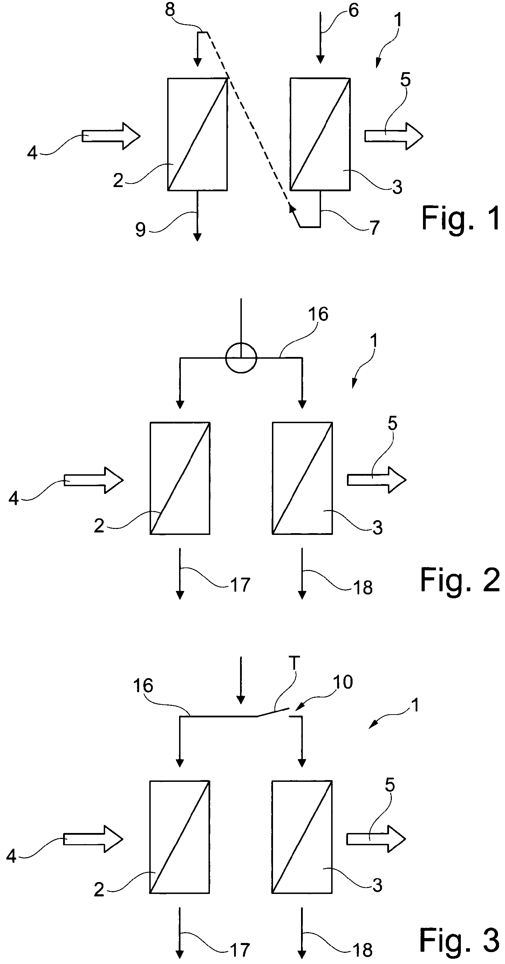

In einer Gas-Austausch-Einheit 1 wird das Blut mit Sauerstoff angereichert und CO2 aus dem Blut abgereichert. Hierzu strömt das Blut durch ein Hohlfasermodul. Das Innere der Hohlfasern (nicht abgebildet) wird mit Gas durchströmt. In Figur 1 werden zwei solcher Hohlfasermodule 2, 3 nacheinander bzw. seriell vom Blut durchströmt. Dabei strömt das Blut hier schematisch dargestellt durch den Pfeil 4 zunächst in die Gas-Austausch-Einheit 2, durchströmt diese sowie dann die Gas-Austausch-Einheit 3, bis es schließlich schematisiert dargestellt durch den Pfeil 5 die Gas-Austausch-Einheit 1 wieder verlässt. Das Gas, dessen Strom durch die Pfeile 6, 7, 8 und 9 schematisiert dargestellt wird, wird, nachdem es den ersten Gastauscher verlassen hat, in den Zweiten eingeleitet. Optional können wie in Figur 2 dargestellt auch beide Gastauscher parallel mit Gas durchströmt werden. So ist es möglich, je nach Bedarf die Gastauschercharakteristik an den Bedarf anzupassen. Das kann bei einer Vorkonfektionierung in der Produktion, vor der Verwendung oder während der Verwendung erfolgen. Der Gasstrom wird hier schematisch durch die Pfeile 16, 17 und 18 abgebildet.

Wie in Figur 1 dargestellt ist ein Gegenlaufen von Blut- und Gasrichtung möglich, wobei das Blut zunächst das Hohlfasermodul 2 und dann das Hohlfasermodul 3 und das Gas zunächst das Hohlfasermodul 3 das Hohlfasermodul 2 durchströmt (sogenanntes Gegenstromprinzip). Auch die umgekehrte Anordnung, bei welcher das Blut zunächst das Hohlfasermodul 2 und dann das Hohlfasermodul 3 durchströmt und das Gas ebenfalls zunächst das Hohlfasermodul 2 das Hohlfasermodul 3 durchströmt, ist möglich (sogenanntes Gleichstromprinzip). Insbesondere ist es möglich, zwischen den beiden Schaltungen - in Serie oder parallel - je nach Bedarf zu wechseln. Wie in Figur 3 abgebildet ist, ist es ebenfalls möglich, eines der beiden Hohlfasermodule zu- oder abzuschalten. Hierzu wird dieses mit einem Ventil 10 verbunden.

Anhand des Ausschnitts aus einem Hohlfasermodul 21 wie in Figur 4 dargestellt soll im Folgenden schematisch die Funktionsweise einer gasseitig vorgeschalteten Befeuchtungs- und Wärmevorrichtung erläutert werden. Bei dieser Anordnung wird das Hohlfasermodul 21 mit erwärmtem Gas und Wasserdampf durchströmt. Der Gasstrom wird durch die Pfeile 22 und 23 symbolisch dargestellt. Der Blutstrom wird durch die Pfeile 24 und 25 symbolisch dargestellt. Die Hohlfasern 27, 28, 29 und 30 sind in zwei Schichten 31 und 32 aus Einbettmaterial fixiert. Das Gas ist mit Wasserdampf gesättigt und weist eine Temperatur über der Bluttemperatur auf. Im Hohlfasermodul 21 gibt das Gas Wärme an das Blut ab und ein Teil des Wasserdampfs kondensiert und gibt die Kondensationsenergie auch an das Blut ab. Das Kondensat 26 wird unterhalb des Hohlfasermoduls 21 gesammelt. Dieses funktioniert daher sowohl als Gas- und Wärmetauscher.

Bei der Gas-Austausch-Einheit 41 sind zwei Hohlfasermodule 42, 43 trommelförmig und konzentrisch ineinander angeordnet. Sie sind durch eine Trennschicht 44 voneinander getrennt, diese kann auch als Gitter oder Netz ausgestaltet sein. Das Blut strömt dabei wie durch die Pfeile 45 und 46 symbolisiert von innen nach außen. Es ist auch möglich, das Blut von außen nach innen oder diagonal nach außen zu führen. Ein möglicher Gasstromverlauf wird erneut durch die Pfeile 48, 49 und 50 symbolisiert. Hierbei wird zunächst das äußere Hohlfasermodul 43 mit Gas beschickt und dann in Serienschaltung das innere Hohlfasermodul 42. Auch eine Parallelschaltung ist hier möglich. Insgesamt stellt sich eine, wie in den Figuren 5 a, b und c aufgebaute Gas-Austausch-Einheit als trommelförmiger Körper dar.

Bei einer Gas-Austausch-Einheit 61 kann, wie in Figur 6 dargestellt, auch eine Stapelanordnung von Hohlfasermodulen 62 und 63 gewählt werden. Die Hohlfasermodule 62, 63 sind dabei als Fasermatten ausgestaltet. Diese können jeweils abwechselnd zueinander gekreuzt angeordnet werden. Das Gas wird symbolisiert durch die Pfeile 66, 67 und 68 jeweils von zwei Seiten durch die Hohlfasern geleitet. Das Blut durchströmt die Gas-Austausch-Einheit 61, wie durch die Pfeile 64, 65 symbolisiert. Zwei oder mehr dieser Einheiten können kombiniert werden und entsprechend der Figuren 1 bis 3 mit Gas beschickt werden.

In Figur 7 a ist die Faseranordnung aus Figur 6 so gestaltet, dass das Einbettmaterial 79 um die Fasermatten wie beispielsweise 78 zur Blutseite einen zylindrischen Abschluss 80 bildet. Bei der Gas-Austausch-Einheit 81 in Figur 7 b sind die Fasermatten 82 und 83 in einem Winkel von 120 ° gekreuzt angeordnet. Hier ist das Einbettmaterial zur Blutseite hin ebenfalls mit einem zylindrischen Abschluss 89 ausgeführt. Die Außenseite ist jedoch im Vergleich zu Figur 7 a nicht quadratisch, sondern hexagonal gestaltet. Der Blutstrom wird durch die Pfeile 84 und 85 symbolisiert, wobei der Gaseinstrom durch die Pfeile 86, 86' und 86" sowie der Gasausstrom durch die Pfeile 87, 87' und 87" symbolisiert wird. Die einzelnen Hohlfasern 88 sind sichtbar.

Bei einer trommelförmigen Faseranordnung 91, 101 wie in Figur 8 a und b dargestellt wird das Blut durch eine blutundurchlässige Schicht 92, 102, 103, 104 spiralförmig von innen nach außen geleitet. Hier ist, wie in der Anordnung 91, ein Kanal, aber auch zwei oder mehrere Kanäle, wie vorliegend drei Kanäle, in der Gas-Austausch-Einheit 101 möglich. Der Bluteinstrom wird hierbei durch die Pfeile 93, 105, 106, 107 symbolisiert, der Blutausstrom durch die Pfeile 94, 108, 109, 110.

Bei der Gas-Austausch-Einheit 121 in Figur 9 a kann ein Teil der Gastauschfasern wie beispielsweise 122, je nach Bedarf durch eine geeignete Ventilanordnung 123 stufenlos zu- oder abgeschaltet werden und somit dem Bedarf angepasst werden. Als Ventil 131 können beispielsweise wie in Figur 9 b dargestellt zwei gegeneinander verschiebbare Lochplatten 132 und 133 verwendet werden. Der Blutstrom wird durch die Pfeile 124 und 125 symbolisiert, der Gasstrom durch die Pfeile 126, 127 und 128.

Wie in Figur 10 dargestellt ist es besonders von Vorteil, wenn die Gas-Austausch-Einheit und die Anordnung der Gastauscherfasern so gewählt wird, dass der Innendurchmesser des blutführenden Bereichs größer ist als dessen Länge. Der Blutstrom wird erneut durch den Pfeil 144 symbolisiert, der Gasstrom durch den Pfeil 145.

Die Gas- Austausch-Einheit 150 in Figur 11 weist zwei Holfasermodule Modul A 151 und Modul B 152 auf, die als Fasermatten ausgebildet sind und hintereinander angeordnet sind. So wird eine Längsaufteilung der Gas- Austausch-Einheit 150 erreicht.

Die Gas- Austausch-Einheit 160, die als Fasermatte ausgebildet ist, weist wie in Figur 12 dargestellt ebenfalls zwei Holfaserbereiche Bereich A 161 und Bereich B 162 auf, die jedoch nebeneinander angeordnet sind. So wird eine Queraufteilung der Gas-Austausch-Einheit 160 erreicht.

Wie in Figur 13 dargestellt kann dann eine unterschiedliche Beschickung Hohlfasermoduls 170 durch Überstromkanalvorrichtungen 171 und 172 erfolgen. Die Zuströmung des Gases erfolgt durch die Zuläufe 173, 174, die jeweils eine zentrale Kammer 175, 178 sowie jeweils zwei Seitenkammern 176, 177 sowie 179, 180 aufweisen. Das Gas strömt von der zentralen Kammer 175, 178 in die zwei Seitenkammern 176, 177 sowie 179, 180. Dadurch wird eine verstärkte Beschickung der Seiten 181 und 182 der Gas-Austauscheinheit 170 im Bereich der zentralen Kammer 175, 178 sowie eine schwächere Beschickung im Bereich der zwei Seitenkammern 176, 177 sowie 179, 180 erreicht. Hierdurch wird eine unterschiedliche Durchströmung der Bereiche A und B wie in Figur 12 dargestellt erzielt. Das Gas verlässt das Faserbündel 187 an den Seiten 183 und 184 wieder und wird durch den Auslass 186 nach draußen geführt.

Diese Anordnung nach Figur 12 des Zulaufs 173 zu der zentralen Kammer 175 mit den Seitenkammern 176, 177 in einer Anordnung ist ebenfalls in Seitenansicht in Figur 14a) und im Ausschnitt eines Querschnittes in Figur 14b) ersichtlich. Hier wird deutlich wie die zentrale Kammer 175 durch Überstromkanäle wie beispielsweise 188 oder 189 mit den Seitenkammern 176, 177 verbunden ist.

Auch in einer Anordnung nach Figur 11 mit einer Beschickung der Hohlfasermodule 206, 207 durch die Überstromkanalvorrichtung 201 wird eine unterschiedliche Beschickung der Hohlfasermodule A 206 und B 207 möglich. Dies ist in Figur 15 in einer Anordnung in Seitenansicht in Figur 15a) und im Ausschnitt eines Querschnittes in Figur 15b) gut ersichtlich. Die Überstromkanalvorrichtung 201 weist einen Zulauf 202 auf, der das Gas in eine erste Kammer 203 einführt von welcher es in eine zweite Kammer 204 durch Überstromkanäle wie beispielweise 205 weitergeleitet wird. Dadurch wird eine unterschiedliche Beschickung der Gas-Austauscheinheit in den Bereichen der beiden Kammern 204,204 im Bereich der unterschiedlichen Hohlfasermodule A 206 und B 207 erreicht.

Eine andere Möglichkeit zur unterschiedlichen Beschickung der Hohlfaserbereiche A und B in einem Aufbau nach Figur 12 ist wie in Figur 16 dargestellt das Aufbringen einer Auflage auf das Faserbündel 211. Hier werden unterschiedliche Auflagen, Auflage A 212, Auflage B 213 auf eine Seitenfläche 215 aufgebracht und dadurch der pneumatische Widerstand erhöht. Der Mittelbereich 214 bleibt folienfrei. Auch hierdurch wird eine unterschiedliche Beschickung der hier drei Bereiche (Faserbereich A im Bereich der Auflage A 212, Faserbereich B 213 im Bereich der Auflage B und Faserbereich C (hier zusätzlich zum Aufbau in Figur 12) im folienfreien Mittelbereich 214.

Eine weitere Möglichkeit zur unterschiedlichen Beschickung der Faserbereiche A und B in einem Aufbau nach Figur 12 ist wie in Figur 16 dargestellt das Aufbringen einer Auflage 222, 223 mit unterschiedlicher Stärke zur pneumatischen Widerstandserhöhung. In Figur 17a) in Seitenaufsicht auf die Auflage 222 in Figur 17b) und Draufsicht auf die Auglage in Figur 17c) sind die Bereiche unterschiedlicher Dicke 225, 226 mit jeweils der Dicke D1 und 224 mit der Dicke D2 erkennbar. Die Auflage 222 ist eine luftdurchlässige Struktur wie beispielsweise ein Flies. Durch die unterschiedliche Dicke wird eine unterschiedliche Gasdurchlässigkeit erreicht, die zu unterschiedlichem Strömungswiderstand führt. Sie wird ebenfalls eine unterschiedliche Beschickung bei der Moduldurchströmung erreicht. Durch eine kontinuierliche Ausgestaltung kann ein kontinuierlicher Beschickungsgradient erreicht werden.

Eine Möglichkeit für einen Aufbau zur variablen Beschickung eines Fasebündels 231 wird wie in Figur 18 dargestellt durch verstellbare Überströmkanäle 232, 233, 234, 235 realisiert. Hierbei ist auf dem Überstromkanalvorrichtung 236, 237 ein Schieber 238, 239 aufgebracht durch welchen die Öffnungen 240, 241, 242, 243, 244, 245 des Überstromkanals 236,237 ganz oder teilweise verdeckt werden können. Hierfür werden die Öffnungen 246, 247, 248, 249, 250, 251 des Schiebers 236, 238 ganz oder teilweise mit denen des Überstromkanals 240, 241, 242, 243, 244, 245 in Deckung mittels einer Verstellvorrichtung 252 gebracht. So kann das Gas entweder ganz oder teilweise direkt in die Kammern des Überstromkanals eintreten oder strömt über die Überströmkanäle 232, 233, 234, 235 in den Kammern weiter.

In einer Gas Austauscheinheit 262 mit einem Faserbündel 262, kann zur Verhinderung der Kondenzwasserbildung im Gehäuse 263 ein elektrisches Heizelement 264 angeordnet sein.

Für die einzelne Hohlfaser ist es von Vorteil, wenn wie in Figur 20 schematisch dargestellt die Faserlänge (L1) zum Faserinnendurchmesser (Di) kleiner als 500_{[OM2]}ist. Insbesondere kann die Länge (L1) kleiner als 80mm sein, der Faserinnendurchmesser (Di) kann in der Größenordnung von 160 bis 200 µm liegen.

## Patentansprüche

1. Gas-Austausch-Einheit (1; 41; 61; 81; 121; 150; 160; 262) mit einem Hohlfasermodul (2, 3, 21; 42, 43), wobei die Gas-Durchström-Charakteristik des Hohlfasermoduls (2, 3, 21; 42, 43) anpassbar ist, und Faserbereiche (A, B, C) des Hohlfasermoduls (2, 3, 21; 42, 43) unterschiedlich mit Gas beschickbar sind, **dadurch gekennzeichnet, dass** unterschiedliche Gase getrennt voneinander zuführbar und abführbar sind.

2. Gas-Austausch-Einheit (1; 41; 61; 81; 121; 150; 160; 262) nach Anspruch 1, **dadurch gekennzeichnet, dass** ein Hohlfasermodul ganz oder teilweise vom Gasstrom zu- oder abschaltbar ist.

3. Gas-Austausch-Einheit (1; 41; 61; 81; 121; 150; 160; 262) nach Anspruch 1 oder 2, **dadurch gekennzeichnet, dass** sie auf der Gasseite eine Blende insbesondere einen Schieber (238, 239) und/oder einen Drehschieber und/oder eine Drossel aufweist, wobei die Blende so angeordnet ist, dass Faserbereiche (A, B, C) nicht durchströmt geschaltet werden können.

4. Gas-Austausch-Einheit (1; 41; 61; 81; 121; 150; 160; 262) nach einem der vorhergehenden Ansprüche, **dadurch gekennzeichnet, dass** an dem Hohlfasermodul eine Überstromkanalvorrichtung (171, 172) angeordnet ist.

5. Gas-Austausch-Einheit (1; 41; 61; 81; 121; 150; 160; 262) nach Anspruch 4, **dadurch gekennzeichnet, dass** die Überstromkanalvorrichtung verstellbare Überstromkanäle (232, 233, 234, 235) aufweist.

6. Gas-Austausch-Einheit (1; 41; 61; 81; 121; 150; 160; 262) nach einem nach einem der vorhergehenden Ansprüche, **dadurch gekennzeichnet, dass** sie eine Abdeckung aufweist.

7. Gas-Austausch-Einheit (1; 41; 61; 81; 121; 150; 160; 262) nach Anspruch 6, **dadurch gekennzeichnet, dass** die Abdeckung einen pneumatischen Widerstandsgradienten aufweist.

8. Gas-Austausch-Einheit (1; 41; 61; 81; 121; 150; 160; 262) nach einem der Ansprüche 1 bis 7, **dadurch gekennzeichnet, dass** dem Hohlfasermodul (2, 3, 21; 42, 43) eine gasseitige Befeuchtungs- und Wärmevorrichtung vorgeschaltet ist.

9. Gas-Austausch-Einheit (1; 41; 61; 81; 121; 150; 160; 262) nach einem der vorhergehenden Ansprüche, **dadurch gekennzeichnet, dass** das Hohlfasermodul (2, 3, 21; 42, 43) trommelförmig ist.

10. Gas-Austausch-Einheit (1; 41; 61; 81; 121; 150; 160; 262) nach einem der vorhergehenden Ansprüche, **dadurch gekennzeichnet, dass** in dem Hohlfasermodul (2, 3, 21; 42, 43) eine blutundurchlässige Schicht (92, 102, 103, 104) von innen nach außen spiralförmig angeordnet ist.

11. Gas-Austausch-Einheit (1; 41; 61; 81; 121; 150; 160; 262) nach einem der vorhergehenden Ansprüche, **dadurch gekennzeichnet, dass** ein Blutstrom von innen nach außen oder von außen nach innen oder diagonal nach außen oder nach innen führbar ist.

12. Gas-Austausch-Einheit (1; 41; 61; 81; 121; 150; 160; 262) nach einem der vorhergehenden Ansprüche, **dadurch gekennzeichnet, dass** sie zwei oder mehr Hohlfasermodule (2, 3, 21; 42, 43) aufweist.

13. Verfahren zum Herstellen einer Gas-Austausch-Einheit (1; 41; 61; 81; 121; 150; 160; 262) nach einem der Ansprüche 1 bis 12, bei welchem in einem ersten Schritt eine automatisierte Herstellung eines Hohlfasermoduls (2, 3, 21; 42, 43) aus einer oder mehreren Fasermatten erfolgt, und bei welchem in einem weiteren Schritt durch Zusammensetzen und Verpotten von mehreren solchen Hohlfasermodulen (2, 3, 21; 42, 43) die Gas-Austausch-Einheit (1; 41; 61; 81; 121; 150; 160; 262) mit unterschiedlicher wirksamer Oberfläche und effektiver Faserlänge modular erzeugt wird.

## Claims

1. Gas exchange unit (1; 41; 61; 81; 121; 150; 160; 262) with a hollow fibre module (2, 3, 21; 42, 43), wherein the gas throughflow characteristic
of the hollow fibre module (2, 3, 21; 42, 43) is adaptable, and fibre regions (A, B, C) of the hollow fibre module (2, 3, 21; 42, 43) can be charged differently with gas, **characterised in that** different gases can be supplied and discharged separately from one another.

2. Gas exchange unit (1; 41; 61; 81; 121; 150; 160; 262) according to claim 1, **characterised in that** a hollow fibre module can be completely or partially connectable to or disconnectable from the gas flow.

3. Gas exchange unit (1; 41; 61; 81; 121; 150; 160; 262) according to claim 1 or 2, **characterised in that** it has a shutter, in particular a slide valve (238, 239) and/or a rotary slide valve and/or a throttle, on the gas side, wherein the shutter is arranged such that fibre regions (A, B, C) cannot be switched to allow flow through.

4. Gas exchange unit (1; 41; 61; 81; 121; 150; 160; 262) according to any of the preceding claims, **characterised in that** an overflow channel device (171, 172) is arranged on the hollow fibre module.

5. Gas exchange unit (1; 41; 61; 81; 121; 150; 160; 262) according to claim 4, **characterised in that** the overflow channel device (171, 172) has adjustable overflow channels (232, 233, 234, 235).

6. Gas exchange unit (1; 41; 61; 81; 121; 150; 160; 262) according to any of the preceding claims, **characterised in that** it has a cover.

7. Gas exchange unit (1; 41; 61; 81; 121; 150; 160; 262) according to claim 6, **characterised in that** the cover has a pneumatic resistance gradient.

8. Gas exchange unit (1; 41; 61; 81; 121; 150; 160; 262) according to one of claims 1 to 7, **characterised in that** a gas-side humidification and heating device is connected upstream of the hollow fibre module (2, 3, 21; 42, 43).

9. Gas exchange unit (1; 41; 61; 81; 121; 150; 160; 262) according to any of the preceding claims, **characterised in that** the hollow fibre module (2, 3, 21; 42, 43) is drum-shaped.

10. Gas exchange unit (1; 41; 61; 81; 121; 150; 160; 262) according to any of the preceding claims, **characterised in that** a blood-impermeable layer (92, 102, 103, 104) is arranged spirally from the inside to the outside in the hollow fibre module (2, 3, 21; 42, 43).

11. Gas exchange unit (1; 41; 61; 81; 121; 150; 160; 262) according to any of the preceding claims, **characterised in that** a blood flow can be guided outwards from inside or inwards from outside or diagonally to outwards or inwards.

12. Gas exchange unit (1; 41; 61; 81; 121; 150; 160; 262) according to any of the preceding claims, **characterised in that** it has two or more hollow fibre modules (2, 3, 21; 42, 43).

13. Method for producing a gas exchange unit (1; 41; 61; 81; 121; 150; 160; 262) according to one of claims 1 to 12, wherein, in a first step, an automated production of a hollow fibre module (2, 3, 21; 42, 43) from one or more fibre mats takes place, and wherein, in a further step, the gas exchange unit (1; 41; 61; 81; 121; 150; 160; 262) with different effective surface area and effective fibre length is generated in a modular manner by combining and potting a plurality of such hollow fibre modules (2, 3, 21; 42, 43).

## Revendications

1. Unité d'échange gazeux (1; 41; 61; 81; 121; 150; 160; 262) comprenant un module à fibres creuses (2, 3, 21; 42, 43), dans laquelle les caractéristiques d'écoulement de gaz du module à fibres creuses (2, 3, 21; 42, 43) sont adaptables, et les zones de fibres (A, B, C) du module à fibres creuses (2, 3, 21; 42, 43) peuvent être alimentées en gaz de manière différenciée, **caractérisée en ce que** différents gaz peuvent être amenés et évacués séparément les uns des autres.

2. Unité d'échange gazeux (1; 41; 61; 81; 121; 150; 160; 262) selon la revendication 1, **caractérisée en ce qu'**un module à fibres creuses peut être entièrement ou partiellement raccordé ou isolé du flux de gaz.

3. Unité d'échange gazeux (1; 41; 61; 81; 121; 150; 160; 262) selon la revendication 1 ou 2, **caractérisée en ce qu'**elle présente, côté gaz, un obturateur, notamment une vanne (238, 239) et/ou une vanne rotative et/ou un organe d'étranglement, l'obturateur étant disposé de sorte que des zones de fibres (A, B, C) puissent être commutées sans être traversées par le flux.

4. Unité d'échange gazeux (1; 41; 61; 81; 121; 150; 160; 262) selon l'une quelconque des revendications précédentes, **caractérisée en ce qu'**un dispositif de canal de dérivation (171, 172) est disposé sur le module à fibres creuses.

5. Unité d'échange gazeux (1; 41; 61; 81; 121; 150; 160; 262) selon la revendication 4, **caractérisée en ce que** le dispositif de canal de dérivation (171, 172) comprend des canaux de dérivation réglables (232, 233, 234, 235).

6. Unité d'échange gazeux (1; 41; 61; 81; 121; 150; 160; 262) selon l'une quelconque des revendications précédentes, **caractérisée en ce qu'**elle comporte un couvercle.

7. Unité d'échange gazeux (1; 41; 61; 81; 121; 150; 160; 262) selon la revendication 6, **caractérisée en ce que** le couvercle présente un gradient de résistance pneumatique.

8. Unité d'échange gazeux (1; 41; 61; 81; 121; 150; 160; 262) selon l'une quelconque des revendications 1 à 7, **caractérisée en ce qu'**un dispositif humidificateur et chauffant côté gaz est installé en amont du module à fibres creuses (2, 3, 21; 42, 43).

9. Unité d'échange gazeux (1; 41; 61; 81; 121; 150; 160; 262) selon l'une quelconque des revendications précédentes, **caractérisée en ce que** le module à fibres creuses (2, 3, 21; 42, 43) est de forme cylindrique.

10. Unité d'échange gazeux (1; 41; 61; 81; 121; 150; 160; 262) selon l'une quelconque des revendications précédentes, **caractérisée en ce qu'**une couche (92, 102, 103, 104) imperméable au sang est disposée en spirale de l'intérieur vers l'extérieur dans le module à fibres creuses (2, 3, 21; 42, 43).

11. Unité d'échange gazeux (1; 41; 61; 81; 121; 150; 160; 262) selon l'une quelconque des revendications précédentes, **caractérisée en ce qu'**un flux sanguin peut être acheminé de l'intérieur vers l'extérieur ou de l'extérieur vers l'intérieur ou en diagonale vers l'extérieur ou vers l'intérieur.

12. Unité d'échange gazeux (1; 41; 61; 81; 121; 150; 160; 262) selon l'une quelconque des revendications précédentes, **caractérisée en ce qu'**elle comprend deux modules à fibres creuses (2, 3, 21; 42, 43) ou plus.

13. Procédé de production d'une unité d'échange gazeux (1; 41; 61; 81; 121; 150; 160; 262) selon l'une quelconque des revendications 1 à 12, dans lequel, dans une première étape, la production automatisée d'un module à fibres creuses (2, 3, 21; 42, 43) est réalisée de manière automatisée à partir d'une ou de plusieurs nappes de fibres, et dans lequel, dans une étape ultérieure, l'unité d'échange gazeux (1; 41; 61; 81; 121; 150; 160; 262) est produite de manière modulaire avec une surface effective et une longueur de fibre effective variables par assemblage et scellement de plusieurs desdits modules à fibres creuses (2, 3, 21; 42, 43) de ce type.
